# EUROPÄISCHE PATENTSCHRIFT

(11) **EP 0 461 518 B1**
(45) Veröffentlichungstag und Bekanntmachung des Hinweises auf die Patenterteilung: **30.11.1994**
(21) Anmeldenummer: 91109077.7
(22) Anmeldetag: 04.06.1991
(51) Int. Cl.: A61M 1/04, A61F 13/14, A61M 27/00

(54) **Ventilpflaster zur Notfallbehandlung offener Thoraxverletzungen**
Plaster with valve for first aid treatment of open thorax injuries
Pansement avec valve pour le traitement d'urgence de blessures ouvertes du thorax

(30) Priorität: 09.06.1990 DE 4018591
(43) Veröffentlichungstag der Anmeldung: 18.12.1991
(73) Patentinhaber: Lohmann GmbH & Co. KG, 56567 Neuwied (DE)
(72) Erfinder: Pollitt, Sebastian, W-5456 Rheinbrohl (DE)
(74) Vertreter: Flaccus, Rolf-Dieter, Dr.

(56) Entgegenhaltungen:
- US-A- 3 419 006
- US-A- 4 465 062
- US-A- 4 813 941

## Beschreibung

Die Erfindung betrifft ein Pflaster zur Notfallbehandlung einer offenen Thoraxverletzung.

Durchgehende Verletzungen des Brustkorbes sind - abgesehen von einer stets gegebenen Infektionsgefahr - deshalb besonders kritisch, weil sie unverzüglich zu einer starken Herabsetzung der Atmung führen können. Ein Grund hierfür besteht zunächst einmal darin, daß der im Thorax normalerweise vorhandene Unterdruck (ca. 4 bis 8 mm Hg) zusammenbricht, da sich aufgrund der Verletzung ein Druckausgleich mit der Umgebung einstellt, so daß der Lungenflügel der verletzten Seite kollabiert.

Eine weitere Beeintrachtigung der Atmung mit dem Lungenflügel der nicht verletzten Seite erfolgt sodann dadurch, daß das zwischen den beiden Lungenflügeln befindliche Mediastinum aufgrund der sich einstellenden Druckverhältnisse zur nicht verletzten Seite hin seitlich verdrängt wird, so daß über den Ausfall des einen Lungenflügels hinaus die Atmung auch noch durch ein reduziertes Atemvolumen des Lungenflügels auf der nicht verletzten Seite reduziert wird.

Weiterhin kommt hinzu, daß der sog. kleine Blutkreislauf in einem solchen Falle so stark belastet wird, daß es zu einer Überbelastung und damit zu einer weiteren ernsthaften Bedrohung für das Leben des Verletzten kommen kann.

Berücksichtigt man dabei, daß Thoraxverletzungen, gemessen an der Gesamtzahl von Unfällen, etwa 9% ausmachen, und daß die Frühmortalität innerhalb der ersten ca. 6 Stunden nach dem betreffenden Unfall sehr hoch ist, so zeigt sich hieraus ohne weiteres, wie schwerwiegend diese Problematik sowohl in qualitativer als auch in quantitativer Hinsicht ist.

Für Erste-Hilfer-Maßnahmen bei derartig offenen Thoraxverletzungen sind verschiedene Möglichkeiten vorgeschlagen worden, die jedoch insgesamt unbefriedigend sind. Diese Vorschläge umfassen beispielsweise den sog. Dachziegelverband, d.h. einen Kompressionsverband, bei dem Heftpflasterstreifen dachziegelartig übereinandergelegt und etwa zu 3/4 um den Thorax gelegt werden, um die Verletzungsstelle notdürftig zu schließen, sowie andere Druckverbände. Für Fälle, in denen kein Verbandzeug zur Hand ist, wird das Auflegen einer flachen Hand auf die Verletzungsstelle und für extreme Situationen wird sogar vorgeschlagen, Lungengewebe der verletzten Seite in den Wundspalt zu ziehen, um die Wunde notdürftig zu schließen. Abgesehen davon, daß eine solche Behandlung ohnehin sehr fraglich ist, kann sie ersichtlich allenfalls von einem Arzt durchgeführt werden.

Eine weitere Möglichkeit der Notversorgung ist die "Vorrichtung zur Notversorgung einer offenen Thoraxverletzung" nach DE 36 31 650 A1.
Sie ist gekennzeichnet durch einen im wesentlichen topfförmigen Hohlkörper aus gasdichtem Material, dessen an die Thorax-Außenseite anzulegender, die Thorax-Verletzung im angelegten Zustand mit Abstand umgebender freier Rand als elastischer, weicher Dichtungsring ausgebildet und mit einer Be- und Entlüftungseinrichtung versehen ist, mittels welcher sein Innenraum unter Schaffung eines Unterdruckes im Innenraum des Hohlkörpers zu belüften ist.

Sämtliche bekannten Maßnahmen ohne Hilfsmittel verhindern allenfalls weitgehend einen weiteren Luftdurchgang durch die Wunde, ergeben aber keine Verbesserung der Atmungsmöglichkeiten. Außerdem ist der Ersthelfer dadurch in der Durchführung weiterer eventuell lebenswichtiger Maßnahmen behindert.

Beim Auflegen bzw. Aufpressen der Hand auf die Verletzungsstelle kann es zudem beim Verletzten zusätzlich zu den ohnedies schon vorhandenen Atembeschwerden zu Angstzuständen kommen.
Die Vorrichtung nach DE 36 31 650 A1 hat den Nachteil, daß sie zu aufwendig und für den Laienhelfer zu kompliziert, also nur schwer anwendbar ist (siehe Medizingeräteverordnung vom 14. Januar 1985, §§ 2 und 6 Abs. 3 und 4).

Aus der US-A 4,465,062 ist eine Vorrichtung für Notfallbehandlung offener Thoraxverletzungen bekannt, bei der in der Öffnung einer mit einer auf der hautzugewandten Seite haftklebenden Beschichtung versehenen Trägerschicht ein Gasrückschlagventil eingelassen ist. Die bekannte Vorrichtung ist vergleichsweise voluminös und dient im wesentlichen zur klinischen Versorgung während oder nach einem operativen Eingriff beispielsweise einer Schußverletzung im Brustraum.

Der vorliegenden Erfindung liegt die Aufgabe zugrunde, eine insbesondere für eine Erste-Hilfe-Maßnahme im Rahmen einer Notversorgung bis zur klinischen Versorgung bestimmte und geeignete Vorrichtung bereitzustellen, die es erlaubt, den Wundspalt nach außen abzuschließen und die Atmungsmöglichkeit des Verletzten schnell und andauernd erheblich zu verbessern. Dabei soll das Pflaster möglichst kompakt sein, um stets einsatzbereit in einem Verbandskasten eines Ersthelfers für eine Notversorgung bis zur klinischen Versorgung mitgeführt zu werden.

Die Lösung der Erfindung gelingt einem Pflaster der im Oberbegriff von Anspruch 1 genannten Art mit der Erfindung dadurch, daß das Gasrückschlagventil ein Membranventil in der Mitte des Trägermaterials ist, das aus einer Kunststoffscheibe mit Löchern besteht, die von einer aufgeklebten Membran so abgedeckt werden, daß diese in der Lage ist, die Löcher luftdicht abzuschließen.
Mit Vorteil ist das Pflaster infolge seiner gedrungenen und kompakten Ausführung für Erste-Hilfe-Maßnahmen bei der Notversorgung einer offenen Thoraxverletzung geeignet, wobei das Pflaster, insbesondere innerhalb seiner Verpackung, stets einsatzbereit in einem Verbandskasten mitgeführt werden kann, so daß es für einen Ersthelfer griffbereit ist. Durch die Ausrüstung mit einem aus einer Kunststoffscheibe bestehenden Membranventil wird erreicht, daß das Pflaster schon bei geringen Druckschwankungen anspricht, wobei es sich während der Einatmungsphase schließt, so daß keine weitere Luft mehr in den Brustkorb eindringen kann. Dagegen öffnet es sich in der Ausatmungsphase, so daß bereits in den Pleuraspalt eingedrungene Luft durch das Ventil entweichen kann.
Der Lungenflügel hat so die Möglichkeit, sich nach jedem Ausatmen wieder etwas weiter zu entfalten, bis er im günstigsten Falle wieder die volle Funktionstüchtigkeit erreicht.

Eine Ausgestaltung sieht vor, daß zur besseren Fixierung des Membranventils von der Unterseite des Trägermaterials ein Ring aus dem gleichen Material wie das Trägermaterial geklebt ist, wobei der Ring halb auf dem Membranventil und halb auf dem Trägermaterial klebt.

Eine Ausgestaltung sieht vor, daß zur Beschichtung des Trägermaterials ein natürlicher Haftkleber wie Kautschukkleber oder ein technischer Haftkleber wie ein Acrylatkleber verwendet ist.

Weitere zweckmäßige Ausgestaltungen des Pflasters sind entsprechend den Unteransprüchen vorgesehen.

Die Erfindung ermöglicht eine einfache und sichere Erste-Hilfe-Maßnahmne bei einer offenen Thoraxverletzung, das Pflaster ist in jeder Situation ohne Zeitverlust und auch durch einen ungeübten Laienhelfer verwendbar. Nach Applikation dieses Pflasters hat der Ersthelfer außerdem die Hände frei, um weitere eventuell lebenserhaltende Erste-Hilfe-Maßnahmen durchführen zu können.
Das Pflaster belastet oder beengt nicht den Betroffenen, wie es z.B. beim Aufpressen der Hand auf den Brustkorb geschieht, so daß das Auftreten von Angstzuständen durch eine derartige Behandlung vermieden werden kann.

Ein Ausführungsbeispiel der Erfindung ist in den Figuren dargestellt und im folgenden näher beschrieben.

Es zeigen:
- Figur 1:: Aufsicht auf das Pflaster mit Membranventil nach abgenommener Membran
- Figur 2:: Querschnitt durch das Ventilpflaster mit aufgeklebter Membran nach Linie I/I der Figur 1.
- Figur 3:: Schematisierte Darstellung des Thorax mit offener Verletzung beim Ausatmen
- Figur 4:: Darstellung gemäß Figur 3 beim Einatmen
- Figur 5:: Darstellung gemäß Figur 3 mit appliziertem Pflaster nach Figur 1 beim Ausatmen
- Figur 6:: Darstellung gemäß Figur 5 beim Einatmen

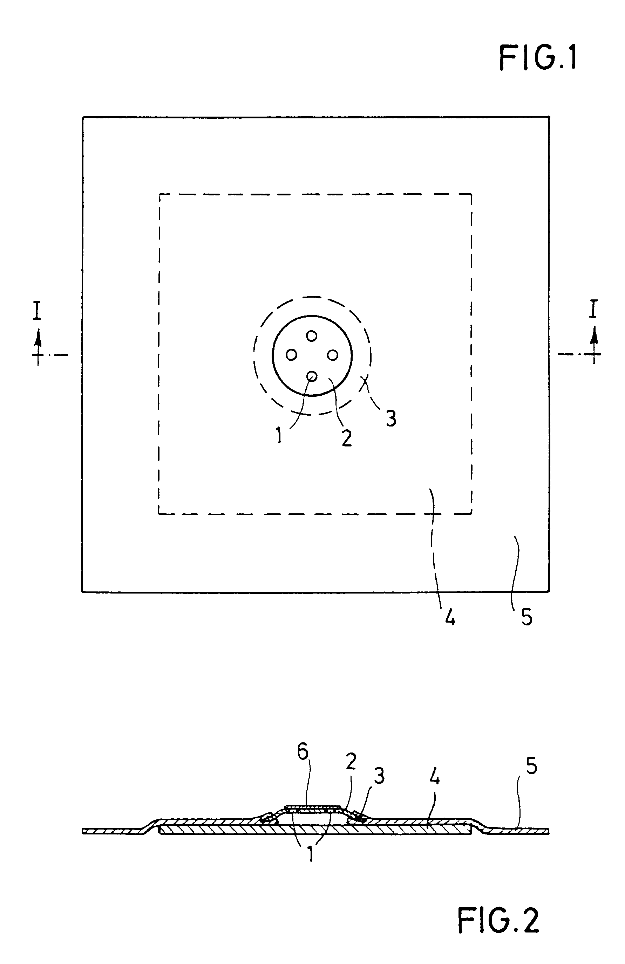

Fig. 1 zeigt ein Ausführungsbeispiel des Ventilpflasters zur Notfallbehandlung offener Thoraxverletzungen in Draufsicht, Fig. 2 einen Querschnitt nach Linie I/I. Das Pflaster weist ein Membranventil 2 in der Mitte des Trägermaterials 5 auf, das aus einer Kunststoffscheibe mit 4 Löchern 1 von 4 mm Durchmesser besteht, die von einer aufgeklebten, in Fig. 2 eingezeichneten Membran abgedeckt werden. Die Membran 6 ist in der Lage, die Löcher 1 luftdicht abzuschließen.
Zur besseren Fixierung des Membranventils 2 wird von der Unterseite des Trägermaterials 5 ein Ring 3 aus dem gleichen Material wie das Trägermaterial 5 geklebt, wobei der Ring 3 halb auf den Membranventil 2 und halb auf dem Trägermaterial 5 klebt.

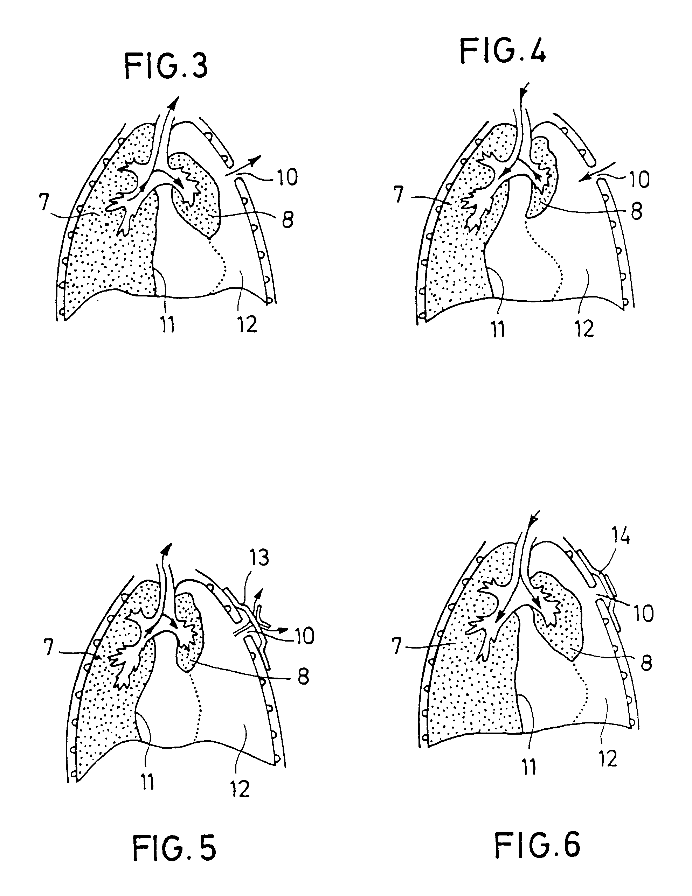

Fig. 3 zeigt in einer schematisierten Darstellung einen eine Thoraxverletzung 10 aufweisenden Thoraxabschnitt im Schnitt. Es ist erkennbar, daß der auf der verletzten Seite liegende Lungenflügel 8 bereits weitgehend kollabiert ist, da der im zugehörigen Thoraxinnenraum 12 herrschende Druck einen Druckausgleich mit der Umgebung erfahren hat, während der andere Lungenflügel 7 bei dem in Fig. 3 dargestellten Ausatmungsvorgang weitgehend normal angeordnet ist, da sich auch das Mediastinum 11 etwa mittig und damit in Normalstellung befindet.

Beim Finatmungsvorgang gemäß Fig. 4 kommt es dagegen zu einer Verdrängung des Mediastiniums in Richtung auf den Lungenflügel 7, wobei der Lungenflügel 8 noch weiter kollabiert.

Wird nun gemäß Fig. 5 ein erfindungsgemäßes Pflaster 13 auf der Außenseite des Thorax appliziert, so daß die Wundauflage 4 über der Verletzung 10 liegt, kommt es beim Ausatmen zu einem geringfügigen Überdruck im Bereich des Thoraxinnenraumes 12.

Dadurch öffnet sich das Ventil 6 und die Luft strömt durch die Löcher 1 nach außen.

Beim Einatmen mit gemäß Fig. 6 appliziertem erfindungsgemäßen Pflaster 14 kommt es zu einem Unterdruck im Thoraxinnenraum, das Ventil schließt sich und der bereits kollabierte Lungenflügel 8 kann sich durch den Unterdruck wieder etwas erholen.
Mit jedem Atemzug wird so eine Verbesserung des Zustandes des Betroffenen herbeigeführt.

## Patentansprüche

1. Pflaster für Erste-Hilfe-Maßnahmen bei der Notversorgung einer offenen Thoraxverletzung mit einer auf der hautzugewandten Seite mit einer haftklebenden Beschichtung versehenen, weitgehend luftundurchlässigen Trägerschicht aus einem textilen Flächengebilde oder aus einer Folie, wobei in der Trägerschicht (5) eine Öffnung vorhanden und in diese ein auf geringe Druckschwankungen ansprechendes Gasrückschlagventil (2) eingelassen ist, welches sich bei dem in der Ausatmungsphase entstehenden Überdruck öffnet und Luft aus dem Brustraum entweichen läßt, während es sich bei dem in der Einatmungsphase entstehenden Unterdrucks schließt, dadurch gekennzeichnet, daß das Gasrückschlagventil ein Membranventil (2) in der Mitte des Trägermaterials (5) ist, das aus einer Kunststoffscheibe mit Löchern (1) besteht, die von einer aufgeklebten Membran (6) so abgedeckt werden, daß diese in der Lage ist, die Löcher (1) luftdicht abzuschließen.

2. Pflaster nach Anspruch 1, dadurch gekennzeichnet, daß zur besseren Fixierung des Membranventils von der Unterseite des Trägermaterials (5) ein Ring (3) aus dem gleichen Material wie das Trägermaterial (5) geklebt ist, wobei der Ring (3) halb auf dem Membranventil (2) und halb auf dem Trägermaterial (5) klebt.

3. Pflaster nach Anspruch 1, dadurch gekennzeichnet, daß zur Beschichtung des Trägermaterials (5) ein natürlicher Haftkleber wie Kautschukkleber oder ein technischer Haftkleber wie ein Acrylatkleber verwendet ist.

4. Pflaster nach einem der vorangehenden Ansprüche, dadurch gekennzeichnet, daß es auf der der Haut zugewandten Seite mit einer ein- oder mehrschichtigen Wundauflage versehen ist.

5. Pflaster nach Anspruch 4, dadurch gekennzeichnet, daß die Wundauflage aus einem luftdurchlässigen, ggf. hochsaugfähigen Material besteht.

6. Pflaster nach Anspruch 4 oder 5, dadurch gekennzeichnet, daß das Material der Wundauflage durch eine entsprechende Oberflächengestaltung gegen Verkleben mit der Wunde geschützt ist, beispielsweise durch Bedampfung mit Aluminium.

7. Pflaster nach Ansprüchen 4 bis 6, dadurch gekennzeichnet, daß die Wundauflage allseitig von der mit einem Haftkleber beschichteten Trägerschicht überragt wird.

8. Pflaster nach einem oder mehreren der Ansprüche 1 bis 7, dadurch gekennzeichnet, daß es in einer geeigneten Verpakkung steril verpackt ist, z.B. in einem Peelbeutel.

## Claims

1. A plaster for first-aid measures in the emergency treatment of open thorax injuries, having a carrier layer made of a textile fabric or of a film, which carrier layer is largely impermeable to air and provided with a pressure-sensitive adhesive coating on the side facing the skin, whereby an aperture is present in said carrier layer (5) and a gas check valve (2) responding to slight pressure variations is inserted in said aperture, which gas check valve (2) opens when positive pressure builds up during the expirium and permits air to escape from the thoracic cavity whereas it closes when negative pressure arises during the inspiratory phase, characterized in that the gas check valve is a diaphragm valve (2) located in the centre of the carrier material (5), which diaphragm valve (2) consists of a plastics disk having holes (1) therein, which holes are covered by a membrane (6) such that said membrane (6) is capable of closing the holes (1) to form an airtight seal.

2. The plaster according to claim 1, characterized in that for better fixation of the diaphragm valve, from the bottom side of the carrier material (5) there is bonded a ring (3) consisting of the same material as the carrier material (5), whereby said ring (3) half adheres to the diaphragm valve (2) and half to the carrier material (5).

3. The plaster according to claim 1, characterized in that a natural pressure sensitive adhesive, such as a rubber adhesive, or a technical pressure sensitive adhesive, such as an acrylic adhesive, is used for coating the carrier material (5).

4. The plaster according to any one of the preceding claims, characterized in that it is provided with a single-layer or multi-layer wound dressing on the side facing the skin.

5. The plaster according to claim 4, characterized in that the wound dressing consists of a material being permeable to air and, optionally, highly absorptive.

6. The plaster according to claim 4 or 5, characterized in that the material of the wound dressing is protected from sticking to the wound by providing it with an appropriate surface design, for example by aluminizing.

7. The plaster according to claims 4 to 6, characterized in that the pressure sensitive adhesive-coated carrier layer projects beyond the wound dressing on all sides.

8. The plaster according to one or more of claims 1 to 7, characterized in that it is packed in a suitable sterile packaging, for example a peel bag.

## Revendications

1. Emplâtre pour la prise de mesures de première aide au cours de soins d'urgence apportés à une blessure ouverte du thorax, avec une couche de support sensiblement imperméable à l'air, pourvue d'un revêtement autocollant du côté faisant face à la peau, en un objet textile plat ou en une feuille, où on a prévu une ouverture dans la couche de support (5), ouverture dans laquelle on a enchâssé un clapet antiretour de gaz (2) sensible à de faibles fluctuations de pression, lequel s'ouvre sous l'effet de la surpression qui prend naissance au cours de la phase d'expiration et laisse l'air s'échapper de la cage thoracique, cependant qu'il se ferme sous l'effet de la dépression qui se crée au cours de la phase d'inspiration, caractérisé en ce que le clapet antiretour de gaz est un clapet à membrane (2) au milieu de la matière de support (5), qui se compose d'un disque en matière plastique pourvu de trous (1) qui sont couverts par une membrane (6) collée sur eux en une manière telle que cette membrane soit en mesure de fermer les trous (1) d'une façon étanche à l'air.

2. Emplâtre selon la revendication 1, caractérisé en ce que, pour assurer une meilleure fixation du clapet à membrane, un anneau (3) en le même matériau que celui de la matière de support (5) est collé depuis la face inférieure de la matière de support (5), si bien que l'anneau (3) colle pour moitié au clapet à membrane (2) et pour moitié à la matière de support (5).

3. Emplâtre selon la revendication 1, caractérisé en ce que, pour le revêtement de la matière de support (5), on utilise une colle de contact naturelle, comme une colle de caoutchouc, ou une colle de contact industrielle, comme une colle d'acrylate.

4. Emplâtre selon l'une quelconque des revendications précédentes, caractérisé en ce qu'il est pourvu, du côté faisant face à la peau, d'un recouvrement de plaie monocouche ou multicouche.

5. Emplâtre selon la revendication 4, caractérisé en ce que le recouvrement de plaie se compose d'un matériau perméable à l'air, éventuellement fortement absorbant.

6. Emplâtre selon la revendication 4 ou 5, caractérisé en ce que le matériau du recouvrement de la plaie est protégé contre une adhérence à la plaie par un façonnement surfacique correspondant, par exemple par déposition d'aluminium.

7. Emplâtre selon l'une quelconque des revendications 4 à 6, caractérisé en ce que le recouvrement de la plaie est surplombé de tous côtés par une couche de support revêtue d'une colle de contact.

8. Emplâtre selon une ou plusieurs des revendications 1 à 7, caractérisé en ce qu'il est emballé dans un conditionnement stérile approprié, par exemple un sachet ou sac à arrachement.
